# EUROPÄISCHE PATENTANMELDUNG

(11) **EP 3 388 408 A1**
(43) Veröffentlichungstag der Anmeldung: **17.10.2018**
(21) Anmeldenummer: 17166077.2
(22) Anmeldetag: 11.04.2017
(51) Int. Cl.: C07C 1/20, C07C 11/167, C07C 45/00, C07C 1/207

(54) **VERFAHREN UND ANLAGE ZUR HERSTELLUNG VON BUTADIEN AUS ETHANOL**

(71) Anmelder: Linde Aktiengesellschaft, 80331 München (DE)
(72) Erfinder: Peschel, Andreas, 82515 Wolfratshausen (DE); Hentschel, Benjamin, 80689 München (DE)
(74) Vertreter: Frommberger, Moritz

(57) **Zusammenfassung**

Die Erfindung betrifft ein Verfahren (100) zur Herstellung von Butadien, bei dem ein Ethanol enthaltender Reaktionseinsatz zu einem ersten Anteil einem ersten Reaktor (1) und zu einem zweiten Anteil einem zweiten Reaktor (2) zugeführt wird, wobei der erste Reaktor (1) mit einem Katalysator ausgestattet ist, der eine direkte Umsetzung von Acetaldehyd mit Ethanol zu Butadien katalysiert und der zweite Reaktor (2) mit einem Katalysator ausgestattet ist, der eine Umsetzung von Ethanol mit Acetaldehyd oder mit Acetaldehyd und Crotonaldehyd zu Butadien katalysiert, wobei dem ersten Reaktor (1) und dem zweiten Reaktor (2) Gasgemische entnommen und zu einem Sammelgemisch vereinigt werden, das neben Butadien zumindest in dem ersten Reaktor gebildetes Acetaldehyd und Crotonaldehyd enthält, und wobei zumindest das Acetaldehyd oder das Acetaldehyd und das Crotonaldehyd zumindest teilweise aus dem Sammelgemisch zurückgewonnen und das zurückgewonnene Acetaldehyd oder das zurückgewonnene Acetaldehyd und das zurückgewonnene Crotonaldehyd zumindest teilweise in den zweiten Reaktor (2) zurückgeführt werden. Eine entsprechende Anlage ist ebenfalls Gegenstand der Erfindung.

## Beschreibung

Die vorliegende Erfindung betrifft ein Verfahren zur Herstellung von Butadien aus Ethanol sowie eine entsprechende Anlage.

### Stand der Technik

1,3-Butadien (nachfolgend auch kurz "Butadien", BD oder 1,3-BD) wird derzeit überwiegend als Nebenprodukt beim Steamcracken von Naphtha gewonnen. Details finden sich beispielsweise bei Bender, M.: "An Overview of Industrial Processes for the Production of Olefins - C4 Hydrocarbons", ChemBioEng Reviews 1, 136-147, 2014. Da jedoch, beispielsweise durch die hohe Verfügbarkeit von kostengünstigem Ethan, statt Naphtha zunehmend auch alternative Einsätze beim Steamcracken verwendet werden, aus denen sich weniger oder kein Butadien bildet, besteht der Bedarf nach Verfahren, die es erlauben, Butadien auch auf anderen Wegen herzustellen. Die vorliegende Erfindung betrifft dabei die Herstellung von Butadien aus Ethanol (EtOH).

Ein Überblick über Eigenschaften, Herstellung und Verwendung von Ethanol findet sich in gängigen Nachschlagewerken, beispielsweise im Artikel "Ethanol" in Ullmann's Encyclopedia of Industrial Chemistry, Onlineveröffentlichung 15. Oktober 2011, DOI: 10.1002/14356007.a09_587.pub2.

Die Herstellung von Ethanol erfolgt derzeit überwiegend fermentativ aus Biomasse. Neben Biomasse kann auch Synthesegas fermentativ zu Ethanol umgesetzt werden. Entsprechende Verfahren werden auch als Gasfermentation bezeichnet und sind beispielsweise bei Brown, R.C.: "Biorenewable Resources: Engineering New Products from Agriculture", Ames, Iowa State Press, 2008, beschrieben.

Die chemische Synthese von Ethanol aus Synthesegas ist ebenfalls bekannt. Sie kann beispielsweise über das Intermediat Methanol erfolgen, das anschließend zu Ethanol und ggf. anderen höheren Alkoholen homologisiert wird. Entsprechende Verfahren sind in der US 4,882,360 A und der US 2013/0123377 A1 offenbart. Eine weitere Route ist die Umsetzung von Methanol zu Essigsäure und die anschließende Hydrierung der Essigsäure zu Ethanol. Beispiele hierfür sind der sogenannte TCX- und der sogenannte SaaBre-Prozess. Die Herstellung von Ethanol durch Carbonylierung von Methanol oder die Hydratisierung von Ethylen ist ebenfalls bekannt.

Auch die chemische Direktsynthese von Ethanol aus Synthesegas, basierend auf Katalysatorsystemen mit den Elementen Rhodium, Ruthenium, Cobalt und/oder Eisen, ist möglich. Ein Überblick über die Katalysatoren findet sich bei Subramani, S.K. und Gangwal: "A Review of Recent Literature to Search for an Efficient Catalytic Process for the Conversion of Syngas to Ethanol", Energy & Fuels 22, 814-839, 2008. Ein entsprechender Katalysator, der relativ selektiv Ethanol und insbesondere kaum höhere Alkohole erzeugt, ist bei Hu et al., "Conversion of Biomass-Derived Syngas to Alcohols and C2 Oxygenates using Supported Rh Catalysts in a Microchannel Reactor", Catalysis Today 120, 90-95, 2007, beschrieben.

Zur Herstellung von Butadien aus Ethanol können das Lebedev- (ETB-L) und das Ostromislensky- Verfahren (ETB-O) eingesetzt werden. Beide Verfahren und die verwendeten Katalysatoren sind in der Fachliteratur, beispielsweise bei Ezinkwo, G.O. et al.: "Fundamental Issues of Catalytic Conversion of Bio-Ethanol into Butadiene", ChemBioEng Reviews 1, 194-203, 2014, beschrieben.

Die Wirtschaftlichkeit und Nachhaltigkeit derartiger Verfahren hängen maßgeblich von der Produktausbeute an Butadien ab. Die vorliegende Erfindung stellt sich daher insbesondere die Aufgabe, eine Verbesserung der Produktausbeute zu erzielen.

### Offenbarung der Erfindung

Diese Aufgabe wird durch ein Verfahren zur parallelen Herstellung von Butadien aus Ethanol und eine entsprechende Anlage mit den Merkmalen der unabhängigen Patentansprüche gelöst. Ausgestaltungen sind jeweils Gegenstand der abhängigen Patentansprüche sowie der nachfolgenden Beschreibung.

### Vorteile der Erfindung

Die vorliegende Erfindung schlägt insbesondere eine Kombination des eingangs erwähnten Lebedev-Verfahrens mit dem ebenfalls eingangs erwähnten Ostromislensky-Verfahren bzw. von dessen Reaktionsschritt gemäß der nachstehenden Reaktionsgleichung (4) vor, wie nachfolgend erläutert.

Beim Lebedev-Verfahren wird ein Katalysator eingesetzt, der Ethanol in einem Schritt zu Butadien, Wasser und Wasserstoff umsetzt:

2 EtOH → 1,3-BD + 2 H₂O + H₂ (1)

Neben den erwähnten Produkten entstehen beim Lebedev-Verfahren eine Reihe an Zwischenkomponenten und Nebenprodukten wie Dieethylether (DEE), Alkene mit zwei bis vier und Alkane mit einem bis vier Kohlenstoffatomen, Aldehyde (vor allem Acetaldehyd, AcAld, und Crotonaldehyd, CrAld), Ketone, Alkohole sowie Ethylacetat.

Die Reaktionsbedingungen des Lebedev-Verfahrens umfassen eine Reaktionstemperatur von 250 bis 450°C (bevorzugt 3 00 bis 400 °C) und einen Überdruck von 1 bis 10 bar (bevorzugt 1 bis 4 bar). Um den Partialdruck der Edukte und Produkte zu reduzieren und damit die Selektivität und die Standzeit des Katalysators zu erhöhen, kann das eingesetzte Ethanol mit einem Verdünnungsmedium (vorzugsweise Dampf) verdünnt werden.

Im Ostromislensky-Verfahren wird Ethanol zunächst zu Acetaldehyd umgesetzt. Dies kann durch Zugabe von Sauerstoff oder durch Dehydrierung erfolgen:

EtOH + 0,5 O₂ → AcAld + H₂O (2)

EtOH → AcAld + H₂ (3)

Die oxidative Reaktion von Ethanol zu Acetaldehyd gemäß Reaktionsgleichung (2) hat dabei den Vorteil, dass ein hoher Umsatz und eine hohe Selektivität erreicht werden. Zudem kann die entstehende Abwärme zur Dampfproduktion genutzt werden. Als Nebenkomponenten werden vor allem Kohlenmonoxid und Kohlendioxid gebildet. Allerdings hat diese auch Nachteile, wie unten erläutert.

Das gemäß den Reaktionsgleichungen (2) oder (3) gebildete Acetaldehyd wird mit Ethanol zu Butadien und Wasser umgesetzt:

EtOH + AcAld →1,3-BD + 2 H₂O (4)

Die beiden Reaktionsstufen gemäß Reaktionsgleichung (2) und (3) einerseits und gemäß Reaktionsgleichung (4) andererseits werden dabei üblicherweise in zwei getrennten Reaktoren durchgeführt.

In der zweiten Reaktionsstufe gemäß Reaktionsgleichung (4) ist das Verhältnis von Ethanol zu Acetaldehyd für die Selektivität wichtig. Hierbei wird Ethanol im Überschuss eingesetzt. Die zweite Reaktion gemäß Reaktionsgleichung (4) wird ansonsten bei ähnlichen Bedingungen wie das Lebedev-Verfahren gemäß Reaktionsgleichung (1) durchgeführt. Eine Literaturübersicht bietet Jones, J.D.: "Catalytic Transformation of Ethanol into 1,3-Butadiene", Chemistry Central Journal 8:53, 2014.

In der zweiten Reaktionsstufe gemäß Reaktionsgleichung (4) treten als Zwischenkomponenten und Nebenprodukte Diethylether, Alkene mit zwei bis vier und Alkane mit einem bis vier Kohlenstoffatomen, Aldehyde (Acetaldehyd, Crotonaldehyd) Ketone, Alkohole (Butanol), Ethylacetat und Verbindungen mit sechs Kohlenstoffatomen auf, also ähnliche Verbindungen wie im Lebedev-Verfahren.

Im Vergleich zu dem im Lebedev-Verfahren eingesetzten Katalysator hat der im Ostromislensky-Verfahren insbesondere für die zweite Reaktionsstufe gemäß Reaktionsgleichung (4) eingesetzte Katalysator eine deutlich höhere Selektivität zu Butadien. Allerdings ist der Ethanolumsatz durch den hohen Überschuss an Ethanol limitiert. Zusätzlich wird der apparative Aufwand deutlich größer, da insgesamt zwei Reaktoren benötigt werden, um die beiden Reaktionsstufen auszuführen.

Im Fall der oxidativen Dehydrierung von Ethanol zu Acetaldehyd gemäß Reaktionsgleichung (2), die aus den oben erläuterten Gründen grundsätzlich vorteilhaft ist, wird zusätzlich reiner Sauerstoff benötigt, wodurch Betriebs- und Investitionskosten steigen. Reste an Sauerstoff müssen dabei unbedingt aus dem Produktgas entfernt werden, da Butadien in der Gegenwart von Sauerstoff zur sogenannten Popcorn-Polymerisation (siehe beispielsweise das "Product Stewardship Guidance Manual" des American Chemistry Council, 2014) neigt und damit Sauerstoff im Prozess ein Sicherheitsrisiko darstellt.

Durch Einsatz eines Ostromislensky-Verfahrens wird damit zwar die Ausbeute an Butadien im Gegensatz zum Lebedev-Verfahren wesentlich erhöht. Allerdings steigen die Investitionskosten signifikant, da ein zusätzlicher Reaktor, eine Sauerstoffversorgung und eine Restentfernung an Sauerstoff aus dem Produktgas oder besondere sicherheitstechnische Maßnahmen benötigt werden. Zusätzlich steigen die Recycleströme durch den reduzierten Umsatz an Ethanol.

Im Rahmen der vorliegenden Erfindung kann dagegen eine Steigerung der Ausbeute an Butadien gegenüber dem Lebedev-Verfahren unter gleichzeitiger Vermeidung des Einsatzes von Sauerstoff erfolgen. Zugleich kommt es im Rahmen der vorliegenden Erfindung zu einem deutlich höheren Ethanolumsatz als im Ostromislensky-Verfahren. Dies wird durch die Kopplung der beiden Verfahren bewirkt.

Die vorliegende Erfindung sieht, wie erwähnt, eine Kombination des Lebedev- mit dem Ostromislensky-Verfahren bzw. mit dessen Reaktionsschritt gemäß Reaktionsgleichung (4) vor. Dazu wird erfindungsgemäß ein Verfahren zur Herstellung von Butadien vorgeschlagen, bei dem ein Ethanol enthaltender Reaktionseinsatz zu einem ersten Anteil einem ersten Reaktor und zu einem zweiten Anteil einem zweiten Reaktor zugeführt wird, wobei der erste Reaktor mit einem Katalysator ausgestattet ist, der eine direkte Umsetzung von Ethanol zu Butadien katalysiert und der zweite Reaktor mit einem Katalysator ausgestattet ist, der eine Umsetzung von Ethanol mit Acetaldehyd oder mit Acetaldehyd und Crotonaldehyd zu Butadien katalysiert.

Ist hier davon die Rede, dass "der erste Reaktor mit einem Katalysator ausgestattet ist, der eine direkte Umsetzung von Ethanol zu Butadien katalysiert", ist damit gemeint, dass der erste Reaktor einen typischen Katalysator aufweist, wie er in einem Lebedev-Verfahren eingesetzt wird. Eine "direkte" Umsetzung ist dabei auf makroskopischer bzw. Reaktorebene zu verstehen, in unmittelbarer Nähe des Katalysators können auch Zwischenschritte auftreten. Es ist jedoch keine Isolierung und gezielte Weiterreaktion von Zwischenprodukten möglich oder vorgesehen. Butadien wird dabei als eines der Hauptprodukte der Reaktion mit einer Selektivität von typischerweise 20 bis 50% gebildet, das Ethanol wird in der Reaktion zu typischerweise 60 bis 90% umgesetzt.

Ist hier ferner davon die Rede, dass "der zweite Reaktor mit einem Katalysator ausgestattet ist, der eine Umsetzung von Ethanol mit Acetaldehyd oder mit Acetaldehyd und Crotonaldehyd zu Butadien katalysiert", ist damit gemeint, dass der zweite Reaktor einen typischen Katalysator aufweist, wie er in einem Ostromislensky-Verfahren zur Durchführung der oben angegebenen Reaktionsstufe gemäß Reaktionsgleichung (4) eingesetzt wird. Auf die Reaktionsstufe gemäß Reaktionsgleichung (2) oder (3) wird hingegen im Rahmen der vorliegenden Erfindung typischerweise verzichtet. Hierbei einsetzbare Katalysatoren sind dem Fachmann auf dem hier einschlägigen Gebiet allgemein bekannt, weshalb auf einschlägige Fachliteratur beispielsweise wie den Artikel "Butadiene" in Ullmann's Encyclopedia of Industrial Chemistry, Onlineausgabe 2005, DOI: 10.1002/14356007.a04_431, den Artikel "Lebedev Process" im Sammelwerk "Comprehensive Organic Name Reactions and Reagents", Onlineveröffentlichung 2010, DOI: 10.1002/9780470638859.conrr387, sowie den Artikel "Ostromislensky Process" in demselben Sammelwerk, Onlineveröffentlichung 2010, DOI: 10.1002/9780470638859.conrr472, verwiesen wird. Der Fachmann wählt einen geeigneten Katalysator nach Bedarf aus.

Erfindungsgemäß werden dem ersten Reaktor und dem zweiten Reaktor Gasgemische entnommen und zu einem Sammelgemisch vereinigt, das neben Butadien zumindest Acetaldehyd und Crotonaldehyd enthält, die in dem ersten Reaktor gebildet bzw. diesem entnommen werden. Wie erwähnt, werden in einem Lebedev-Verfahren bzw. bei Verwendung eines entsprechenden Katalysators als Nebenprodukte entsprechende Aldehyde, insbesondere Acetaldehyd und Crotonaldehyd, gebildet. Acetaldehyd alleine, oder aber Acetaldehyd und Crotonaldehyd, wird bzw. werden erfindungsgemäß zumindest teilweise aus dem Sammelgemisch zurückgewonnen und zumindest teilweise in den zweiten Reaktor zurückgeführt. Auf diese Weise kann, wie bereits erwähnt, auf die Reaktionsstufe gemäß Reaktionsgleichung (2) oder insbesondere (3) eines Ostromislensky-Verfahrens verzichtet werden, weil der Bedarf an Acetaldehyd durch die erwähnte Rückführung gedeckt werden kann. Ist der zweite Reaktor mit einem Katalysator ausgestattet, der neben Acetaldehyd auch zur Umsetzung von Crotonaldehyd in der Lage ist, kann auch das Crotonaldehyd, mit denselben Vorteilen, zumindest teilweise in den zweiten Reaktor zurückgeführt werden. Ist dies nicht der Fall, kann Crotonaldehyd auch zumindest teilweise in den ersten Reaktor zurückgeführt werden. Auch eine bedarfsgerechte, anteilige Rückführung des oder der Aldehyde in den ersten und zweiten Reaktor gleichzeitig ist möglich.

Es versteht sich, dass zunächst auch mehrere Aldehyde, insbesondere Acetaldehyd und Crotonaldehyd gemeinsam, zumindest teilweise aus dem Sammelgemisch abgetrennt, aber nur eines der Aldehyde, insbesondere Acetaldehyd, zumindest teilweise in den zweiten Reaktor zurückgeführt werden kann. Ferner versteht sich, dass wenn "ein" Aldehyd zurückgeführt wird, ein entsprechend rückgeführter Strom auch Restanteile eines weiteren Aldehyds aufweisen kann.

Crotonaldehyd wird als wichtige Zwischenkomponente bei der Bildung von Butadien sowohl im Lebedev-Verfahren gemäß der obigen Reaktionsgleichung (1) angesehen. Die hierbei am Katalysator (wie erwähnt ist die "direkte" Umsetzung von Butadien zu Ethanol auf makroskopischer ebene zu verstehen) ablaufenden Reaktionen umfassen eine Reaktion von Ethanol zu Acetaldehyd und eine Weiterreaktion von Acetaldehyd mit weiterem Ethanol zu Crotonaldehyd. Das Crotonaldehyd reagiert mit Wasserstoff zu Butadien und Wasser. Die genannten Reaktionen laufen im Lebedev-Verfahren an einem Katalysator (mit vergleichsweise niedriger Selektivität ab). Gerade die Bildung von Crotonaldehyd und die Weiterreaktion von Crotonaldehyd zu Butadien erfolgt vergleichsweise langsam. Daher ist Crotonaldehyd, aber auch Acetaldehyd, beim Lebedev-Verfahren bzw. Verwendung eines entsprechenden Katalysators als Nebenkomponenten am Reaktoraustritt vorhanden. Bei der zweiten Stufe des Ostromislenski-Verfahrens gemäß der obigen Reaktionsgleichung (4) erfolgt am Katalysator ebenfalls zumindest eine Reaktion von Acetaldehyd mit Ethanol zu Crotonaldehyd. Hier ist die Bildung von Crotonaldehyd und die Weiterreaktion von Crotonaldehyd zu Butadien aber deutlich schneller, weshalb hier höhere Selektivitäten erreicht werden und typischerweise kein oder zumindest deutlich weniger Aldehyde am Reaktoraustritt vorhanden sind.

Auf diese Weise lassen sich die Vorteile des Ostromislensky-Verfahrens, die insbesondere in einer erhöhten Ausbeute an Butadien im Gegensatz zum Lebedev-Verfahren besteht, nutzen, ohne dass dessen Nachteile in Kauf genommen werden müssen. So sind insbesondere kein entsprechender zusätzlicher Reaktor zur Durchführung der Reaktionen gemäß den Reaktionsgleichungen (2) bzw. (3), keine dort ansonsten erforderliche Sauerstoffversorgung und keine Restentfernung an Sauerstoff aus dem Produktgas oder besondere sicherheitstechnische Maßnahmen aufgrund vorhandenen Sauerstoffs erforderlich.

Vorteilhafterweise werden im Rahmen der vorliegenden Erfindung der Katalysator und die Reaktionsbedingungen in dem ersten Reaktor derart gewählt, dass in diesem ein Ethanolumsatz von 60 bis 90% und/oder eine Selektivität zu Butadien von 20 bis 70% und/oder eine Selektivität zu Acetaldehyd von 10 bis 20% und/oder eine Selektivität zu Crotonaldehyd von 0,1 bis 5% erzielt werden. Entsprechende Werte lassen sich unter Verwendung eines zuvor beschriebenen Katalysators erzielen, wie er typischerweise in einem Lebedev-Verfahren eingesetzt wird.

Vorteilhafterweise werden im Rahmen der vorliegenden Erfindung ferner der Katalysator und die Reaktionsbedingungen in dem zweiten Reaktor derart gewählt, dass in diesem ein Ethanolumsatz von 20 bis 70% und/oder eine Selektivität zu Butadien von 60 bis 80% erzielt werden. Entsprechende Werte lassen sich unter Verwendung eines zuvor beschriebenen Katalysators erzielen, wie er typischerweise in einem Ostromislensky-Verfahren zur Durchführung der Reaktion gemäß der obigen Reaktionsgleichung (4) eingesetzt wird.

Gemäß einer vorteilhaften Ausführungsform werden dem ersten Reaktor und dem zweiten Reaktor ferner jeweils Dampf als Verdünnungsmittel zugeführt. Auf diese Weise lässt sich die Konzentration der Edukte und Produkte im Sinne einer verbesserten Reaktionskinetik reduzieren.

Der erste und der zweite Anteil des Reaktionseinsatzes werden vorteilhafterweise in Abhängigkeit von einer Menge des oder der in den zweiten Reaktor zurückgeführten Aldehyde und insbesondere einem vorgegebenen Verhältnis von Ethanol zu dem oder den Aldehyden, insbesondere zu Acetaldehyd, das typischerweise oberhalb von 1 mol/mol liegt, eingestellt. Auf diese Weise lässt sich ein Produktspektrum je nach Bedarf entsprechend verschieben.

Gemäß einer besonders vorteilhaften Ausführungsform der Erfindung umfasst die zumindest teilweise Rückgewinnung des oder der Aldehyde aus dem Sammelgemisch, das Sammelgemisch oder ein aus diesem gebildetes Sekundärgemisch einer Kaltwasserwäsche mit Wasser auf einem Temperaturniveau von 0 bis 15°C zu unterwerfen. Auf diese Weise können das oder die Aldehyde auf besonders einfache und effiziente Weise aus einem entsprechenden Gasgemisch abgetrennt und zurückgewonnen werden. Die tiefen Temperaturen des Waschwassers in der Kaltwasserwäsche erhöhen dabei eine Selektivität zu den genannten Aldehyden und damit die Ausbeute.

Vorteilhafterweise wird diese Kaltwasserwäsche dabei auf einem Druckniveau von 5 bis 50 bar durchgeführt. Auf diese Weise lässt sich die Größe der verwendeten Apparate verringern und die Ausbeute gegenüber niedrigeren Druckniveaus, auf denen die Reaktoren betrieben werden, erhöhen.

Insbesondere wird im Rahmen der vorliegenden Erfindung der Kaltwasserwäsche ein aus dem Sammelgemisch gebildetes Sekundärgemisch unterworfen, wobei die Bildung des Sekundärgemischs umfasst, das Sammelgemisch einer Wasservorwäsche auf einem Druckniveau von 1 bis 5 bar und einer anschließenden Verdichtung zu unterwerfen. Sowohl bei der Wasservorwäsche als auch bei der Verdichtung werden insbesondere schwerere Komponenten aus dem Sammelgemisch abgeschieden. Diese können unter Erhalt einer Wasserfraktion zurückgewonnen werden, aus der Dampf erzeugt werden kann, der zusammen mit dem ersten und dem zweiten Anteil des Reaktionseinsatzes in den ersten bzw. zweiten Reaktor eingespeist werden kann.

Ein in der Kaltwasserwäsche verbleibendes Gasgemisch wird vorteilhafterweise einer Butadienextraktion unterworfen, in der das gebildete Butadien insbesondere unter Verwendung eines Waschmittels, das N-Methyl-2-Pyrrolidon und/oder Dimethylformamid enthält, zurückgewonnen wird.

Die vorliegende Erfindung erstreckt sich auch auf eine Anlage zur Herstellung von Butadien, die Mittel aufweist, die dafür eingerichtet sind, einen Ethanol enthaltenden Reaktionseinsatz zu einem ersten Anteil einem ersten Reaktor und zu einem zweiten Anteil einem zweiten Reaktor zuzuführen, wobei der erste Reaktor mit einem Katalysator ausgestattet ist, der eine direkte Umsetzung von Ethanol zu Butadien katalysiert und der zweite Reaktor mit einem Katalysator ausgestattet ist, der eine Umsetzung von Ethanol mit Acetaldehyd oder mit Acetaldehyd und Crotonaldehyd zu Butadien katalysiert, wobei ferner Mittel vorgesehen sind, die dafür eingerichtet sind, dem ersten Reaktor und dem zweiten Reaktor Gasgemische zu entnehmen und zu einem Sammelgemisch zu vereinigen, das neben Butadien zumindest in dem ersten Reaktor gebildete Aldehyde, darunter Acetaldehyd und Crotonaldehyd, enthält, und wobei Mittel vorgesehen sind, die dafür eingerichtet sind, das Acetaldehyd oder das Acetaldehyd und das Crotonaldehyd zumindest teilweise aus dem Sammelgemisch zurückzugewinnen und in den zweiten Reaktor zurückzuführen.

Vorteilhafterweise ist eine entsprechende Anlage zur Durchführung eines Verfahrens eingerichtet, wie es zuvor erläutert wurde, und weist entsprechende Mittel auf. Auf die oben erläuterten Merkmale und Vorteile wird daher ausdrücklich verwiesen.

Die Erfindung wird nachfolgend unter Bezugnahme auf die beigefügte Zeichnung näher erläutert, die eine Ausführungsform der Erfindung näher veranschaulicht.

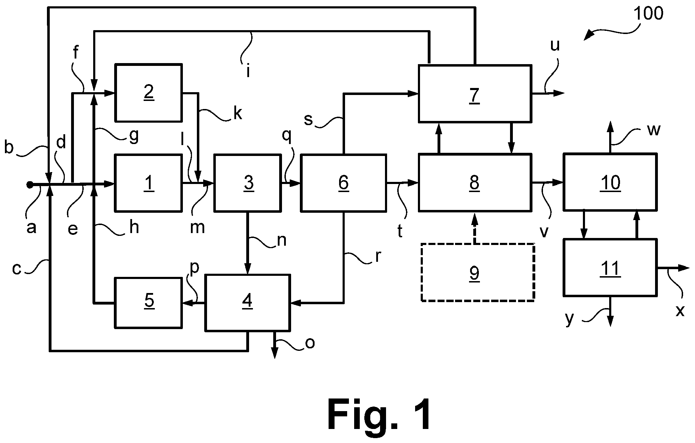

Kurze Beschreibung der Zeichnungen
- Figur 1: veranschaulicht ein Verfahren gemäß einer Ausführungsform der Erfindung.

### Ausführliche Beschreibung der Zeichnungen

In Figur 1 ist ein Verfahren gemäß einer Ausführungsform der Erfindung veranschaulicht und insgesamt mit 100 bezeichnet.

Mit 1 bis 11 sind dabei sowohl Verfahrensschritte als auch zu deren Realisierung verwendete technische Einrichtungen bezeichnet. Die Erläuterungen zu Figur 1 betreffen damit eine entsprechende Anlage in gleicher Weise.

In dem Verfahren 100 wird beispielsweise unter Verwendung technisch reinen Ethanols oder einer insbesondere azeotropen Mischung, insbesondere von Ethanol und Wasser, das bzw. die in Form eines Stoffstroms a bereitgestellt wird, und zwei rückgeführten Stoffströmen b und c ein Reaktionseinsatz in Form eines Stoffstroms d gebildet.

Der Reaktionseinsatz wird bei Bedarf verdampft und in zwei Teilströme e und f aufgeteilt. Der Reaktionseinsatz wird in Form der Teilströme e und f zu einem ersten Anteil einem ersten Reaktor 1 und zu einem zweiten Anteil einem zweiten Reaktor 2 zugeführt. Der erste Reaktor 1 ist zur direkten Umsetzung von Ethanol zu Butadien eingerichtet und weist einen entsprechenden Katalysator auf, der zweite Reaktor 2 ist zur Umsetzung von Ethanol mit Acetaldehyd und/oder Crotonaldehyd zu Butadien eingerichtet und weist einen entsprechenden Katalysator auf. Der erste Reaktor 1 ist also zur Durchführung einer Reaktion gemäß der eingangs angegebenen Reaktionsgleichung (1) eines Lebedev-, der zweite Reaktor 2 zur Durchführung einer Reaktion gemäß der eingangs angegebenen Reaktionsgleichung (4) eines Ostromislensky-Verfahrens eingerichtet.

Beiden Anteilen des Reaktionseinsatzes und damit den Teilströmen e und f wird Prozessdampf in Form der Stoffströme g und h zugegeben, um die Edukt- und Produktkonzentration in den Reaktoren 1 und 2 zu reduzieren. Um Missverständnisse bei Betrachtung der Figur 1 auszuschließen, sei betont, dass der Stoffstrom g Prozessdampf, aber keinen Anteil des Teilstroms e umfasst. Ferner wird dem Teilstrom f stromauf des zweiten Reaktors 2 ein rückgeführter, Acetaldehyd und Crotonaldehyd enthaltender Stoffstrom i zugespeist.

Das Verhältnis, in dem der Reaktionseinsatz dem ersten Reaktor 1 einerseits und dem zweiten Reaktor 2 andererseits zugeführt wird, wird dabei in Abhängigkeit von einer Menge des Acetaldehyds und Crotonaldehyds des Stoffstroms i und insbesondere einem vorgegebenen Verhältnis von Ethanol zu Acetaldehyd, das typischerweise oberhalb von 1 mol/mol liegt, gewählt.

Im ersten Reaktor 1 werden typischerweise ein Ethanolumsatz von ca. 60 bis 90%, eine Selektivität zu Butadien von ca. 20 bis 70%, eine Selektivität zu Acetaldehyd von ca. 10 bis 20% und eine Selektivität zu Crotonaldehyd von ca. 0,1 bis 5% erzielt (zu den Nebenprodukten sei auch hier auf den eingangs zitierten Stand der Technik verwiesen). Im zweiten Reaktor 2 werden typischerweise ein Ethanolumsatz von ca. 20 bis 70% und eine Selektivität zu Butadien von ca. 60 bis 80% erzielt (zu den Nebenprodukten sei auf den eingangs zitierten Stand der Technik verwiesen).

Den Reaktoren 1, 2 in Form der Produktströme k und I entnommenen Gasgemische werden zu einem Sammelstrom m bzw. einem Sammelgemisch vereinigt. Der Sammelstrom m wird einer Wasservorwäsche 3 zugeführt, wo der Sammelstrom m abgekühlt und teilweise von schwereren Komponenten befreit wird. wird. Sich in der Wasservorwäsche 3 abscheidendes Kondensat wird als Stoffstrom n einer Wasserregenerierung 4 zugeführt und in dieser von organischen Komponenten befreit. Dabei rückgewonnenes Ethanol wird in Form des bereits erwähnten Stoffstroms c rückgeführt. Abwasser wird in Form eines Stoffstroms o aus dem Verfahren 100 ausgeführt, Teile des Kondensats in Form eines Stoffstroms p in eine Prozessdampferzeugung 5 geführt.

Die Gasphase aus der Wasservorwäsche 3 wird in Form eines Stoffstroms q in einer Rohgasverdichtung 6 verdichtet. Diese wird i.d.R. mehrstufig mit Zwischenkühlung ausgeführt. Dabei fallen wässrige und organische Kondensate an. Die wässrigen Kondensate werden in Form eines Stoffstroms r in die Wasserregenerierung 4 eingespeist, die organischen Kondensate in Form eines Stoffstroms s einer Aldehydrückgewinnung 7 zugeführt.

Der in der Rohgasverdichtung 6 gasförmig verbleibende Rest wird in Form eines Stoffstroms t einer Kaltwasserwäsche 8 zugeführt und dort von Aldehyden, insbesondere Acetaldehyd, aber auch ggf. Crotonaldehyd, und weiteren organischen Komponenten befreit. Das in der Kaltwasserwäsche 8 eingesetzte Wasser wird dabei vergleichsweise tief vorgekühlt (unter Kühlwasserniveau, z.B. mit Hilfe eines C3-Kältemittels auf ca. 10 °C), um die Selektivität un d die Kapazität des Wassers für Acetaldehyd und ggf. Crotonaldehyd zu erhöhen. Ein entsprechendes Kühlsystem 9 und zugeführte Prozesskälte sind gestrichelt veranschaulicht. Ein in der Kaltwasserwäsche 8 erhaltener beladener Wasserstrom (in Figur 1 nicht gesondert bezeichnet) wird in der Aldehydrückgewinnung 7 regeneriert und wieder in die Kaltwasserwäsche 8 zurückgeführt.

Acetaldehyd und Crotonaldehyd sind Zwischenprodukte und können in Form des bereits erwähnten Stoffstroms i rückgeführt werden. Auch eine Rückführung von Acetaldehyd alleine oder andere Ausgestaltungen sind, wie bereits oben erwähnt, grundsätzlich möglich. Die Aldehyde werden in der Aldehydrückgewinnung 7 von den restlichen organischen Komponenten abgetrennt, die in Form eines Stoffstroms u aus dem Verfahren 100 ausgeführt werden. Der Stoffstrom u umfasst typischerweise zumindest Ethylacetat, Methylethylketon, Aceton, Butanol und andere Kohlenwasserstoffe.

Ein verbleibender Gasstrom v enthält neben Butadien noch weitere Verbindungen mit vier Kohlenstoffatomen, leichte Gase wie Wasserstoff, Kohlenmonoxid, Ethylen, Propylen, Diethylether sowie Verbindungen mit fünf und sechs Kohlenstoffatomen. Das Wertprodukt Butadien wird dabei mit einem geeigneten Extraktionsverfahren (z.B. auf Basis von N-Methyl-2-Pyrrolidon oder Dimethylformamid) in einer Butadienextraktion 10 aus dem Gasstrom abgetrennt. Dabei entsteht ein Reststrom w an leichten Gasen, der thermisch oder stofflich in einem anderen Prozess verwertet werden kann. In dem beladenen Lösemittel (nicht gesondert bezeichnet), das in eine Regenerierung 11 überführt wird, finden sich neben Butadien, das in Form eines Stoffstroms x aus dem Verfahren 100 ausgeführt wird, auch Diethylether und höhere Kohlenwasserstoffe, die in Form eines oder mehrerer Stoffströme y aus dem Verfahren ausgeführt werden. Das regenerierte Lösemittel wird wieder der Butadienextraktion 10 zugeführt.

## Patentansprüche

1. Verfahren (100) zur Herstellung von Butadien, bei dem ein Ethanol enthaltender Reaktionseinsatz zu einem ersten Anteil einem ersten Reaktor (1) und zu einem zweiten Anteil einem zweiten Reaktor (2) zugeführt wird, wobei der erste Reaktor (1) mit einem Katalysator ausgestattet ist, der eine direkte Umsetzung von Ethanol zu Butadien katalysiert und der zweite Reaktor (2) mit einem Katalysator ausgestattet ist, der eine Umsetzung von Ethanol mit Acetaldehyd oder mit Acetaldehyd und Crotonaldehyd zu Butadien katalysiert, wobei dem ersten Reaktor (1) und dem zweiten Reaktor (2) Gasgemische entnommen und zu einem Sammelgemisch vereinigt werden, das neben Butadien zumindest in dem ersten Reaktor gebildetes Acetaldehyd und Crotonaldehyd enthält, und wobei das Acetaldehyd oder das Acetaldehyd und das Crotonaldehyd zumindest teilweise aus dem Sammelgemisch zurückgewonnen und das zurückgewonnene Acetaldehyd oder das zurückgewonnene Acetaldehyd und das zurückgewonnene Crotonaldehyd zumindest teilweise in den zweiten Reaktor (2) zurückgeführt werden.

2. Verfahren (100) nach Anspruch 1, bei dem der erste Reaktor (1) zur Durchführung des Lebedev-Verfahrens, bei dem Ethanol zu Butadien umgesetzt wird, eingerichtet ist, und bei dem der zweite Reaktor (2) zur Durchführung eines Reaktionsschritts des Ostromislensky-Verfahrens, in dem Ethanol mit Acetaldehyd oder Acetaldehyd und Crotonaldehyd zu Butadien umgesetzt wird, eingerichtet ist.

3. Verfahren (100) nach Anspruch 2, bei dem aus dem Sammelgemisch das Acetaldehyd zumindest teilweise zurückgewonnen und zumindest teilweise in den den zweiten Reaktor (2) zurückgeführt wird.

4. Verfahren (100) nach Anspruch 3, bei dem aus dem Sammelgemisch ferner das Crotonaldehyd zumindest teilweise zurückgewonnen und zumindest teilweise in den den zweiten Reaktor (2) zurückgeführt wird.

5. Verfahren (100) nach Anspruch 3, bei dem aus dem Sammelgemisch ferner das Crotonaldehyd zumindest teilweise zurückgewonnen und zumindest teilweise in den den ersten Reaktor (1).

6. Verfahren (100) nach einem der vorstehenden Ansprüche, bei dem der Katalysator und die Reaktionsbedingungen in dem ersten Reaktor (1) derart gewählt werden, dass in diesem ein Ethanolumsatz von 60 bis 90% und/oder eine Selektivität zu Butadien von 20 bis 50% und/oder eine Selektivität zu Acetaldehyd von 10 bis 20% und/oder eine Selektivität zu Crotonaldehyd von 0,1 bis 5% erzielt werden.

7. Verfahren (100) nach einem der vorstehenden Ansprüche, bei dem der Katalysator und die Reaktionsbedingungen in dem zweiten Reaktor (2) derart gewählt werden, dass in diesem ein Ethanolumsatz von 20 bis 70% und/oder eine Selektivität zu Butadien von 60 bis 80% erzielt werden.

8. Verfahren (100) nach einem der vorstehenden Ansprüche, bei dem dem ersten Reaktor (1) und dem zweiten Reaktor (2) ferner jeweils Dampf zugeführt werden.

9. Verfahren (100) nach einem der vorstehenden Ansprüche, bei dem die zumindest teilweise Rückgewinnung des oder der Aldehyde aus dem Sammelgemisch umfasst, das Sammelgemisch oder ein aus diesem gebildetes Sekundärgemisch einer Kaltwasserwäsche (8) mit Wasser auf einem Temperaturniveau von 0 bis 15 °C zu unterwerfen.

10. Verfahren nach Anspruch 9, bei dem die Kaltwasserwäsche (8) auf einem Druckniveau von 5 bis 50 bar durchgeführt wird.

11. Verfahren (100) nach einem der Ansprüche 9 oder 10, bei dem der Kaltwasserwäsche (8) das aus dem Sammelgemisch gebildete Sekundärgemisch unterworfen wird, wobei die Bildung des Sekundärgemischs umfasst, das Sammelgemisch einer Wasservorwäsche (3) auf einem Druckniveau von 1 bis 5 und einer anschließenden Verdichtung (6) zu unterwerfen.

12. Verfahren (100) nach Anspruch 11, bei dem bei der Wasservorwäsche (3) und der Verdichtung (6) Komponenten aus dem Sammelgemisch abgeschieden werden.

13. Verfahren (100) nach einem der Ansprüche 9 bis 12, bei dem ein nach der Kaltwasserwäsche (8) verbleibendes Gasgemisch einer Butadienextraktion (10) unterworfen wird.

14. Verfahren (100) nach Anspruch 10, bei dem in der Butadienextration (10) ein Waschmittel verwendet wird, das N-Methyl-2-Pyrrolidon und/oder Dimethylformamid enthält.

15. Anlage zur Herstellung von Butadien, die Mittel aufweist, die dafür eingerichtet sind, einen Ethanol enthaltenden Reaktionseinsatz zu einem ersten Anteil einem ersten Reaktor (1) und zu einem zweiten Anteil einem zweiten Reaktor (2) zuzuführen, wobei der erste Reaktor (1) mit einem Katalysator ausgestattet ist, der eine direkte Umsetzung von Ethanol zu Butadien katalysiert und der zweite Reaktor (2) mit einem Katalysator ausgestattet ist, der eine Umsetzung von Ethanol mit Acetaldehyd oder mit Acetaldehyd und Crotonaldehyd zu Butadien katalysiert, wobei ferner Mittel vorgesehen sind, die dafür eingerichtet sind, dem ersten Reaktor (1) und dem zweiten Reaktor (2) Gasgemische zu entnehmen und zu einem Sammelgemisch zu vereinigen, das neben Butadien zumindest in dem ersten Reaktor gebildetes Acetaldehyd und Crotonaldehyd enthält, und wobei Mittel vorgesehen sind, die dafür eingerichtet sind, das Acetaldehyd oder das Acetaldehyd und das Crotonaldehyd zumindest teilweise aus dem Sammelgemisch zurückzugewinnen und das zurückgewonnene Acetaldehyd oder das zurückgewonnene Acetaldehyd und das zurückgewonnene Crotonaldehyd in den zweiten Reaktor (2) zurückzuführen.
